# EUROPEAN PATENT APPLICATION

(11) **EP 4 624 482 A1**
(43) Date of publication of application: **01.10.2025**
(21) Application number: 23895010.9
(22) Date of filing: 21.11.2023
(51) Int. Cl.: C07F 9/6571, A61K 31/665, A61P 25/28

(54) **PYRIMIDOPYRIMIDINONE COMPOUND AND PHARMACEUTICAL COMPOSITION COMPRISING SAME**

(30) Priority: 23.11.2022 KR 20220158569
(71) Applicant: Axceso Biopharma Co., Ltd., Anyang-si, Gyeonggi-do 14056 (KR)
(72) Inventor: KIM, Joon Woo, Yongin-si, Gyeonggi-do 17003 (KR); KIM, Su Jin, Siheung-si, Gyeonggi-do 14923 (KR); PARK, Young Jun, Hoengseong-gun, Gangwon-do (KR); CHOI, Myeong Jun, Yongin-si, Gyeonggi-do 16963 (KR)
(74) Representative: V.O.
(86) International application number: PCT/KR2023/018824
(87) International publication number: WO 2024/112083

(57) **Abstract**

The present disclosure relates to a phytosphingosine-3,4-cyclicphosphate compound and a pharmaceutical composition containing the compound as an active ingredient, which can increase the expression of SIRT1 and decrease the expression of amyloid and Tau proteins, as well as increase the expression of tyrosine hydroxylase and decrease the expression of α-synuclein, and ameliorate sepsis and acute respiratory distress syndrome induced by CLP surgery.

## Description

### [Technical Field]

The present disclosure relates to a phytosphingosine-3,4-cyclicphosphate compound and pharmaceutical composition comprising the same, and more specifically to a phytosphingosine-3,4-cyclicphosphate compound capable of increasing SIRT1 expression, decreasing the expression of amyloid and Tau proteins, increasing tyrosine hydroxylase expression, decreasing α-synuclein expression, as well as ameliorating sepsis and acute respiratory distress syndrome induced by CLP surgery, and pharmaceutical composition comprising the same as an active ingredient.

### [Background Art]

Neurodegenerative diseases are literally any of the degenerative diseases that occur in the brain as we age, which are caused by the loss of nerve cells. Neurodegenerative diseases include Alzheimer's disease (AD), Parkinson's disease (PD), Huntington's disease (HD), multiple sclerosis (MS), and amyotrophic lateral sclerosis (ALS), also known as Lou Gehrig's disease, with Alzheimer's disease being the most common, followed by Parkinson's disease.

Alzheimer's disease is the main neurodegenerative disease that causes senile dementia and is characterized by progressive memory and cognitive loss, while Parkinson's disease is caused by the death of dopaminergic neurons in the substantia nigra pars compacta (SNpc) and dopamine deficiency in the striatum, resulting in symptoms such as tremor, bradykinesia, muscle stiffness, postural instability, and akinesia.

Both Alzheimer's disease and Parkinson's disease have been identified as being characterized by abnormal aggregation of proteins. Abnormal aggregation of extracellular amyloid beta (Aβ) and intracellular Tau in Alzheimer's disease and α-synuclein in Parkinson's disease is known to be closely related to the pathophysiology of the diseases.

For example, Korean Patent No. 10-1064258 discloses a composition for preventing degeneration and damage of brain cells caused by amyloid beta, comprising an arylureidoacetate compound, and Korean Patent No. 10-1092620 discloses a composition for preventing or treating neurodegenerative diseases by inhibiting the expression and modification of α-synuclein, which causes damage and death of nerve cells, comprising a sesquiterpene compound as an active ingredient.

Recently, it has been reported that the expression of SIRT1 (silent mating type information regulation 2 homolog; sirtuin 1) is reduced in these neurodegenerative diseases, and SIRT1 activators have emerged as therapeutic agents for neurodegenerative diseases.

Meanwhile, sepsis is an inflammatory reaction induced by excessive activation of the body's immune system by infection with pathogenic microorganisms. In severe cases, it leads to shock and death in the patient.

Specifically, sepsis is a systemic inflammatory reaction syndrome caused by infection and mainly occurs acutely in infants, the elderly, or surgical patients with weak immunity. Sepsis is mostly accompanied by a systemic inflammatory response, and systemic inflammatory response can be caused by various causes. A systemic inflammatory response caused by infection with pathogenic microorganisms in the body is called sepsis. Sepsis is a disease that accounts for the majority of deaths in critically ill patients, and is a common infectious disease accounting for about 25-30% of hospitalized patients. Sepsis is a dangerous condition with a mortality rate of 30-60%, but there is no effective treatment.

Various attempts are being made to develop a therapeutic agent in the absence of an effective therapeutic agent for sepsis. In particular, many attempts have been made as a method of inhibiting inflammation, but various anti-inflammatory drugs often fail in clinical practice, so a new treatment strategy is needed rather than a method to inhibit inflammation.

Recently, it was found that the activity and synthesis of SIRT1 protein and the content of sphingosine-1-phosphate (S1P) were decreased in sepsis patients compared to the normal group (Critical care, 2015, 19, 372). S1P content tends to decrease sharply in sepsis patients, so an attempt is being made to treat sepsis by using an S1P agonist to increase the S1P content or by using an inhibitor that inhibits an enzyme that degrades S1P (J Pharmacol Exp Ther., 2015, 352, 61-66), and attempts are being made to increase SIRT1 protein content and activity decreased in sepsis patients. The best way to increase the content of S1P is to overproduce the gene that synthesizes S1P or knock down or knock out the enzyme that degrades S1P, but this method is inconvenient due to genetic manipulation, and methods using S1P agonists have shown little therapeutic benefit.

In addition, severe sepsis can lead to acute respiratory distress syndrome (ARDS).

ARDS is a syndrome characterized by acute onset pulmonary edema that causes dyspnea that does not improve with high levels of oxygen. In addition to sepsis, this syndrome can be caused by trauma, massive blood transfusions, and medications. ARDS is a dangerous condition with a mortality rate of 40-50%, but there is no effective treatment.

### [Disclosure]

### [Technical Problem]

An object of the present disclosure is to provide a compound represented by the following formula (1), or pharmaceutically acceptable salt thereof, which exhibits preventive, therapeutic and ameliorating effects on neurodegenerative diseases, sepsis and acute respiratory distress syndrome.

Another object of the present invention is to provide a pharmaceutical composition comprising a compound represented by the above formula (1) or pharmaceutically acceptable salt thereof.

Yet another object of the present invention is to provide a dietary supplement comprising a compound represented by the above formula (1) or pharmaceutically acceptable salt thereof.

### [Technical Solution]

One aspect of the present invention relates to a compound represented by the following formula (1) or pharmaceutically acceptable salt thereof. 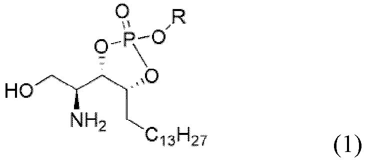 wherein,
R is hydrogen, a C₁-C₆ alkyl or an aryl.

The term "C₁-C₆ alkyl" as used herein means a straight or branched monovalent hydrocarbon having 1 to 6 carbon atoms, and examples include, but are not limited to, methyl, ethyl, n-propyl, i-propyl, n-butyl, i-butyl, t-butyl, n-pentyl, n-hexyl, and the like.

The term "aryl" as used herein includes both aromatic and heteroaromatic groups and partially reduced derivatives thereof. The aromatic group is a simple or fused ring type composed of 5 to 15 carbon atoms, and the heteroaromatic group is an aromatic group containing one or more of oxygen, sulfur, or nitrogen. Representative examples of the aryl include, but are not limited to, phenyl, naphthyl, pyridinyl, pyrimidinyl, furanyl, thiophenyl, indolyl, quinolinyl, imidazolinyl, oxazolyl, thiazolyl, tetrahydronaphthyl, and the like.

In one embodiment of the present invention, R is hydrogen, or a C₁-C₆ alkyl.

The pharmaceutically acceptable salt of the present invention may include both non-toxic inorganic and organic acid salts, and examples thereof include hydrochloride, sulfate, nitrate, phosphate, acetate, adipate, aspartate, benzoate, benzenesulfonate, citrate, camphorate, camphorsulfonate, diphosphate, ethanesulfonate, fumarate, glutamate, malate, lactate, methanesulfonate, succinate, tartrate, picrate, tosylate, and the like. In particular, it may be a hydrochloride salt of a compound represented by formula (1), which is represented by the following formula (1a).

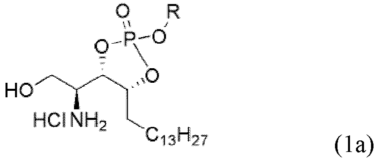

In another aspect, the present invention provides a method for preparing a compound represented by formula (1) or pharmaceutically acceptable salt thereof comprising:
(i) protecting an amine group of a compound represented by the following formula (2) to obtain a compound represented by the following formula (3);
(ii) protecting a primary hydroxyl group of a compound represented by the following formula (3) to obtain a compound represented by the following formula (4);
(iii) reacting the compound represented by the following formula (4) with phosphoryl chloride (POCl₃), or phosphoryl chloride (POCl₃) and a compound represented by the following formula (5), or a compound represented by the following formula (6), to obtain a compound represented by the following formula (7);
(iv) selectively deprotecting the hydroxyl protecting group of the compound represented by the following formula (7) to obtain a compound represented by the following formula (8); and
(v) deprotecting the amine protecting group of the compound represented by the following formula (8).

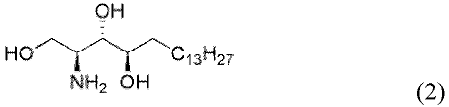

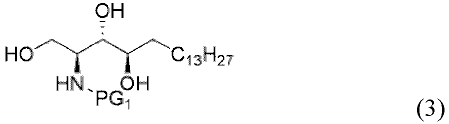

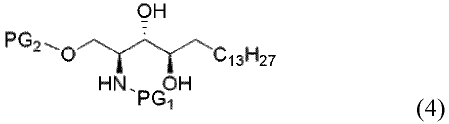

   R'-OH (5) 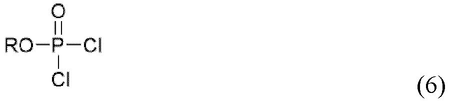 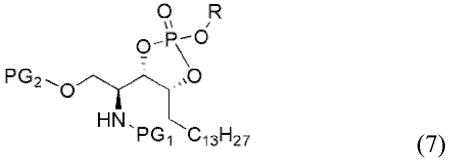 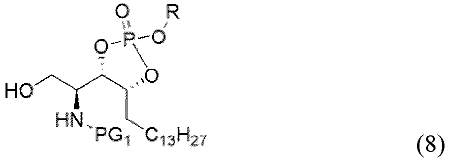 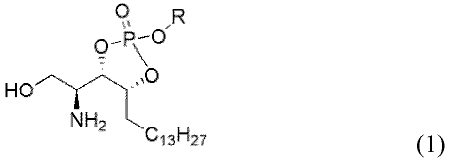
wherein,
PG₁ is an amine protecting group;
PG₂ is a hydroxyl protecting group;
R' is a C₁-C₆ alkyl or an aryl; and
R is hydrogen, a C₁-C₆ alkyl or an aryl.

In one embodiment of the invention, the amine protecting group may be, but is not limited to, t-butyloxycarbonyl, benzyloxycarbonyl, and the like.

In one embodiment of the invention, the hydroxyl protecting group may be, but is not limited to, t-butyldimethylsilyl, pivaloyl, and the like.

Hereinafter, a preparation method of the present invention will be described in more detail with reference to the following reaction scheme (1). The method described in the following reaction scheme (1) is an example of a representative method, and the reaction reagents, reaction conditions, etc. may be changed at any time depending on the case.

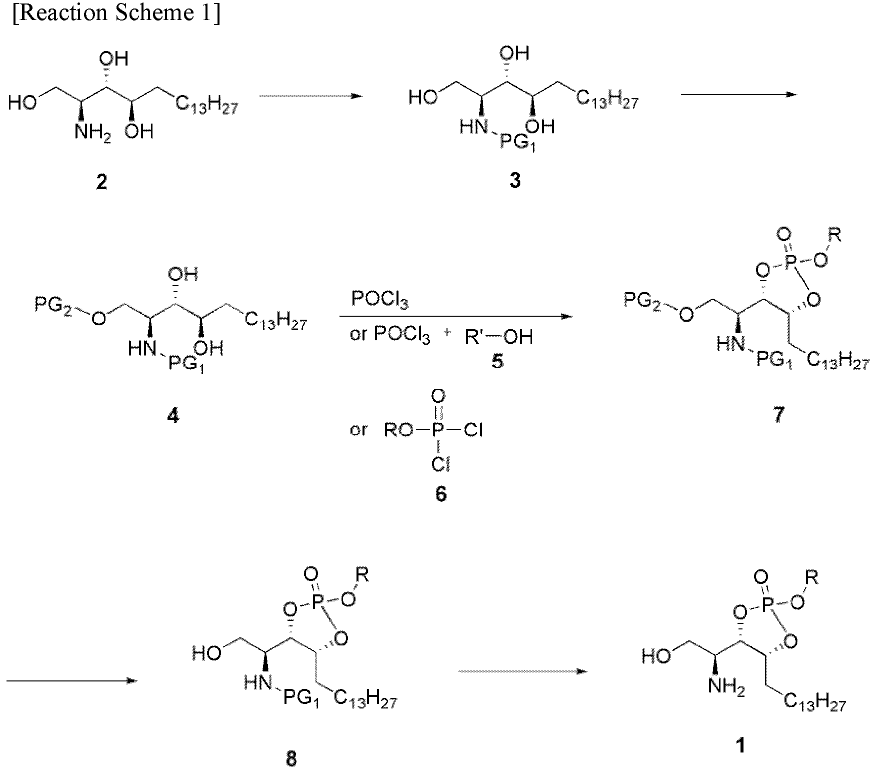

### Step 1: Synthesis of a compound represented by formula (3)

A compound represented by formula (3) can be prepared by protecting the amine group of a compound represented by formula (2).

The protection may be performed using di-t-butyl dicarbonate (Boc₂O), benzyl chloroformate, or the like.

### Step 2: Synthesis of a compound represented by formula (4)

A compound represented by formula (4) can be prepared by protecting the primary hydroxyl group of a compound represented by formula (3).

The protection may be performed using t-butyl dimethylsilyl chloride, pivaloyl chloride, or the like in the presence of a catalyst and a base.

In this case, 4-dimethylaminopyridine or the like may be used as the catalyst, and triethylamine, imidazole, or the like may be used as the base.

### Step 3: Synthesis of a compound represented by formula (7)

A compound represented by formula (7) can be prepared by reacting a compound represented by formula (4) with phosphoryl chloride (POCl₃), or phosphoryl chloride (POCl₃) and a compound represented by formula (5), or a compound represented by formula (6).

The reaction may be performed in the presence of a base, in which the base may be pyridine or the like.

### Step 4: Synthesis of a compound represented by formula (8)

A compound represented by formula (8) can be prepared by selectively deprotecting the hydroxyl protecting group of the compound represented by formula (7).

The deprotection may be performed in the presence of a base and HF.

As the base, pyridine or the like may be used.

### Step 5: Synthesis of a compound represented by formula (1)

A compound represented by formula (1) can be prepared by deprotecting the amine protecting group of the compound represented by formula (8).

The deprotection may be performed using an acid.

In this case, the acid may be an inorganic acid or an organic acid, for example hydrochloric acid.

By performing deprotection using an acid as described above, acid addition reaction is also possible along with deprotection, so that the compound represented by formula (1) can be obtained in the form of an acid addition salt.

The compound represented by formula (1) or pharmaceutically acceptable salt thereof according to the present invention can increase the expression of SIRT1 and can restore reduced intestinal length in animal models of neurodegenerative diseases (see Experimental Examples 1 and 2).

Furthermore, the compound represented by formula (1) or pharmaceutically acceptable salt thereof according to the present invention can reduce the expression of amyloid and Tau proteins, which are biomarkers of Alzheimer's disease (see Experimental Example 3).

In addition, the compound represented by formula (1) or pharmaceutically acceptable salt thereof according to the invention can exhibit neuronal proliferation potency in Parkinson's disease cell models, promote the differentiation of pluripotent stem cells into dopaminergic neurons, as well as restore the expression of TH decreased by rotenone and conversely decrease the expression of α-synuclein increased by rotenone (see Experimental Examples 4 to 6).

Moreover, the compound represented by formula (1) or pharmaceutically acceptable salt thereof according to the present invention can ameliorate sepsis and acute respiratory distress syndrome (ARDS) induced by CLP surgery (see Experimental Examples 7 and 8).

Furthermore, the compound represented by formula (1) or pharmaceutically acceptable salt thereof according to the present invention can ameliorate sepsis and acute respiratory distress syndrome through inhibition of inflammation and strengthening of the vascular barrier (see Experimental Examples 9 and 10).

Thus, another aspect of the present invention relates to a pharmaceutical composition for preventing or treating a neurodegenerative disease comprising a compound represented by formula (1) or pharmaceutically acceptable salt thereof.

Specifically, the neurodegenerative disease may be Alzheimer's disease, Parkinson's disease, or the like.

Further, yet another aspect of the present invention relates to a pharmaceutical composition for preventing or treating sepsis or acute respiratory distress syndrome comprising a compound represented by formula (1) or pharmaceutically acceptable salt thereof.

The pharmaceutical composition according to the present invention may be administered orally (e.g., by taking or inhaling) or parenterally (e.g., by injection, deposition, implantation, suppository), and the injection may be, for example, intravenous, subcutaneous, intramuscular, or intraperitoneal. Depending on the route of administration, the pharmaceutical composition according to the present invention may be formulated as tablets, capsules, granules, fine subtilae, dispersions, sublingual tablets, suppositories, ointments, injections, emulsions, suspensions, syrups, sprays, and the like. The pharmaceutical composition according to the present invention in various forms described above can be prepared by methods known in the art using pharmaceutically acceptable carriers conventionally used for each formulation. Examples of pharmaceutically acceptable carriers include excipients, binders, disintegrating agents, lubricants, preservatives, antioxidants, isotonic agents, buffers, coatings, sweeteners, solubilizers, bases, dispersants, wetting agents, suspending agents, stabilizers, colorants, and the like.

A pharmaceutical composition according to the present invention comprises from about 0.001 to 95% by weight of a compound of the present invention or pharmaceutically acceptable salt thereof, depending on the form of the drug.

The specific dosage of the pharmaceutical composition of the present invention may vary depending on the type of mammal, including human, being treated, body weight, sex, severity of the disease, and the judgment of the physician. Preferably, in the case of oral administration, 0.01 to 50 mg of the active ingredient per kilogram of body weight per day is administered, and in the case of parenteral administration, 0.001 to 10 mg of the active ingredient per kilogram of body weight per day is administered. The total daily dose may be administered in one or several doses, depending on the severity of the disease, the physician's judgment, etc.

Another aspect of the present invention relates to a dietary supplement for preventing or ameliorating a neurodegenerative disease comprising a compound represented by the above formula (1) or pharmaceutically acceptable salt thereof.

Specifically, the neurodegenerative disease may be Alzheimer's disease, Parkinson's disease, or the like.

The type of dietary supplement according to the present invention is not particularly limited and can be in the form of oral preparations such as powders, granules, tablets, capsules, suspensions, emulsions, syrups, or added to common foods such as candies, confectionery, chewing gum, ice cream, noodles, bread, beverages, etc.

The dietary supplement of the present invention may be prepared by conventional methods, depending on the form, with fillers, extenders, binders, wetting agents, disintegrating agents, sweeteners, flavoring agents, preservatives, surfactants, lubricants, excipients, and the like, as appropriate.

In the preparation of the above dietary supplement, the content of the compound represented by formula (1) or pharmaceutically acceptable salt thereof depends on the form of the dietary supplement, but is from about 0.001 to 10 wt%, preferably from 0.01 to 5 wt%.

### [Advantageous Effects]

The compound according to the present invention can increase the expression of SIRT1 and restore reduced intestinal length in animal models of neurodegenerative diseases. Furthermore, the compound according to the present invention can reduce the expression of amyloid and Tau proteins, which are biomarkers of Alzheimer's disease, show proliferative efficacy of nerve cells in a cell model of Parkinson's disease, promote the differentiation of pluripotent stem cells into dopaminergic neurons, as well as restore the expression of TH decreased by rotenone and, conversely, reduce the expression of α-synuclein increased by rotenone. In addition, the compound according to the present invention can ameliorate sepsis and acute respiratory distress syndrome induced by CLP treatment.

Thus, the compound according to the present invention can be effectively used in compositions for preventing, treating and ameliorating neurodegenerative diseases, sepsis and acute respiratory distress syndrome.

### [Brief Description of Figures]

FIG. 1 shows western blot results measuring changes in the expression of SIRT1 upon treatment with a compound of formula (1a-1) in HUVEC cells.
FIG. 2 shows results of intestinal length changes upon treatment with a compound of formula (1a-1) in an AD-induced animal model.
FIG. 3 shows fluorescence microscopy results of changes in the expression of AD biomarkers mAβ and Tau upon treatment with a compound of formula (1a-1) in an AD-induced animal model.
FIG. 4 shows results of evaluating the proliferation efficacy of nerve cells upon treatment with a compound of formula (1a-1) in a cell model of Parkinson's disease.
FIG. 5 shows results of evaluating the efficacy in promoting differentiation of pluripotent stem cells into dopaminergic neurons upon treatment with a compound of formula (1a-1).
FIG. 6 shows western blot results measuring changes in the expression of tyrosine hydroxylase and α-synuclein upon treatment with a compound of formula (1a-1) and a graph depicting them.
FIG. 7 is a graph showing the effect on the survival rate of mice when a compound of formula (1a-1) of 100 ng/mouse (5 µg/kg) was administered at 6 and 16 hours after CLP treatment.
FIG. 8 is a graph showing the effect on the survival rate of mice when fingolimod (FTY720) of 100 ng/mouse (5 µg/kg) was administered at 6 and 16 hours after CLP treatment.
FIG. 9A is a photograph of lung tissue observed under an optical microscope when treated with a compound of formula (1a-1) after CLP treatment, FIG. 9B is a graph showing the results of the lung injury score, which evaluates lung tissue damage using a histological score, and FIG. 9C is a graph showing the wet-to-dry weight ratio of the harvested lungs.
FIG. 10 is a graph showing the effect of treatment with a compound of formula (1a-1) on the secretion of the inflammatory cytokines TNF-α (A) and IL-6 (B) after CLP treatment.
FIG. 11A is a graph showing the effect on the permeability of the MYSEC cell lines upon LPS treatment in the presence of a compound of formula (1a-1), and FIGS. 11B and 11C are a photograph and a graph, respectively, showing the effect on the permeability of mouse organs upon treatment with a compound of formula (1a-1) after CLP treatment.

### [Best Model

Hereinafter, the present invention is further illustrated by the following examples. These examples are intended to illustrate the present invention only, and it will be apparent to those skilled in the art that the scope of the present invention is not limited to these examples.

### Example 1: Preparation of (4S,5R)-4-((S)-1-amino-2-hydroxyethyl)-2-hydroxy-5-tetradecyl-1,3,2-dioxaphosphoran-2-oxide hydrochloride (1a-1)

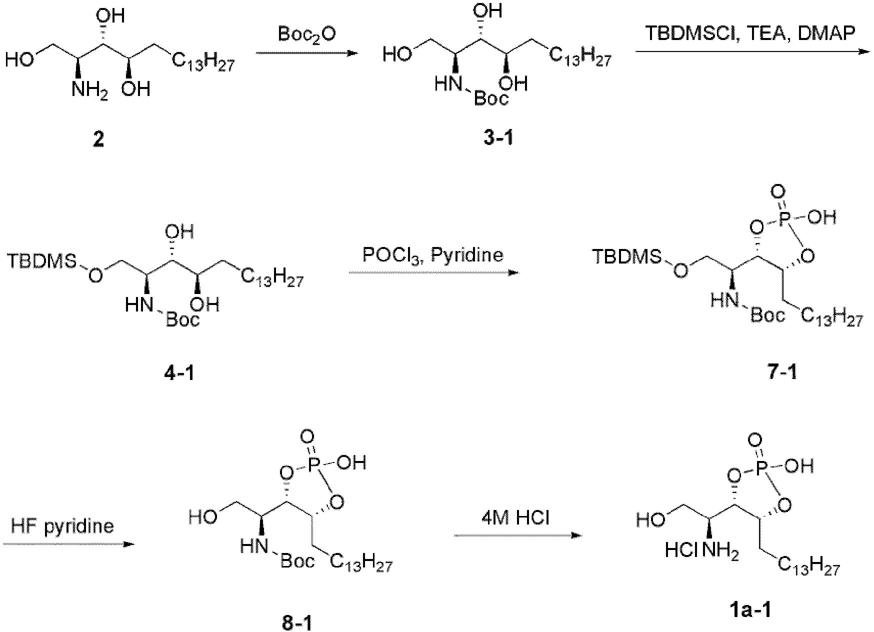

### Example 1-1: Preparation of t-butyl ((2S,3S,4R)-1,3,4-trihydroxyoctadecan-2-yl)carbamate (3-1)

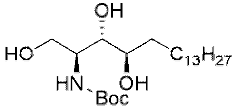

DS-phytosphingosine (2) 100 g (314.951 mmol) was dissolved in 1000 mL of tetrahydrofuran, di-t-butyl dicarbonate (Boc₂O) 82.5 g (377.941 mmol) was added thereto, and the mixture was stirred for 18 hours. After the reaction was completed, the insoluble material was filtered through filter paper and concentrated under reduced pressure. After adding 800 mL of hexane to the concentrated oil phase and stirring, the resulting crystals were filtered and vacuum dried to give 118.36 g (90%) of the title compound.
¹H NMR (DMSO-d₆): δ 0.81-0.85(3H, t), 1.21-1.30(24H, m), 1.35-1.40(9H, m), 1.45-1.53(2H, m), 3.31-3.41(2H, m), 3.46-3.54(2H, m), 4.40-4.42(1H, m)
ES-MS m/z: 418.44 [M+H]⁺

### Example 1-2: Preparation of t-butyl ((2S,3S,4R)-1-((t-butyldimethylsilyl)oxy)-3,4-dihydroxyoctadecan-2-yl)carbamate (4-1)

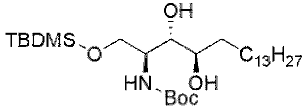

5 g (11.973 mmol) oft-butyl ((2S,3S,4R)-1,3,4-trihydroxyoctadecan-2-yl)carbamate (3-1) obtained in Example 1-1 above was dissolved in 50 mL of dichloromethane, t-butyl dimethylsilyl chloride 2 g (13.170 mmol), 4-dimethylaminopyridine 0.73 g (5.986 mmol), and triethylamine 1.85 mL (13.170 mmol) were added thereto, and the mixture was stirred for 16 hours. After the reaction was completed, 30 mL of water was added. The separated organic layer was dried over anhydrous magnesium sulfate and concentrated under reduced pressure. The obtained material was purified using column chromatography to give 6.33 g (99%) of the title compound.
¹H NMR (DMSO-d₆): δ 0.81-0.85(12H, m), 1.13-1.27(24H, m), 1.34-1.39(9H, m), 1.45-1.51(2H, m), 3.23-3.41(2H, m), 3.61-3.64(1H, m), 3.74-3.77(1H, dd), 3.98-4.03(1H, m)

### Example 1-3: Preparation of t-butyl ((1S)-2-((t-butyldimethylsilyl)oxy)-1-((4S,5R)-2-hydroxy-2-oxido-5-tetradecyl-1,3,2-dioxaphosphoran-4-yl)ethyl)carbamate (7-1)

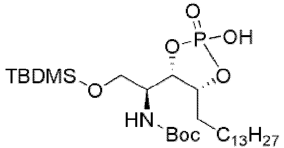

9.3 g (17.472 mmol) of t-butyl ((2S,3S,4R)-1-((t-butyldimethylsilyl)oxy)-3,4-dihydroxyoctadecan-2-yl)carbamate (4-1) obtained in Example 1-2 above was dissolved in 80 mL of dichloromethane and then cooled to 0 °C. After adding 8.5 mL (104.832 mmol) of pyridine, 2.1 mL (22.714 mmol) of phosphoryl trichloride diluted in 20 mL of dichloromethane was slowly added dropwise. The mixture was stirred at 20-25 °C for 3 hours, and after the reaction was completed, 50 mL of water was added to extract the compound. The separated organic layer was dried over anhydrous magnesium sulfate and concentrated under reduced pressure to give 10.3 g (100%) of the title compound.
¹H NMR (DMSO-d₆): δ 0.88-0.90(12H, m), 1.27-1.33(24H, m), 1.42-1.45(9H, m), 1.55-1.63(2H, m), 3.46-3.54(2H, m), 3.66-3.74(1H, m), 4.41-4.51(2H, m)
ES-MS m/z: 594.62 [M+H]⁺

### Example 1-4: Preparation of t-butyl ((1S)-2-hydroxy-1-((4S,5R)-2-hydroxy-2-oxido-5-tetradecyl-1,3,2-dioxaphosphoran-4-yl)ethyl)carbamate (8-1)

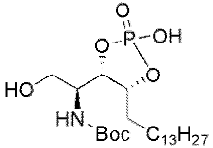

10.3g (17.472 mmol) of t-butyl ((1S)-2-((t-butyldimethylsilyl)oxy)-1-((4S,5R)-2-hydroxy-2-oxido-5-tetradecyl-1,3,2-dioxaphosphoran-4-yl)ethyl)carbamate (7-1) obtained in Example 1-3 above was dissolved in 100 mL of tetrahydrofuran, then 1.8 mL (69.888 mmol) of hydrogen fluoride pyridine was added, and the mixture was stirred at 20-25 °C for 16 hours. After the reaction was completed, 7.4 g (69.888 mmol) of sodium carbonate was added and stirred for 3 hours. After adding 50 mL of 1N hydrochloric acid to the reaction solution, 100 mL of ethyl acetate was added to perform extraction. The separated organic layer was dried over anhydrous magnesium sulfate and concentrated under reduced pressure to give 6 g (71.6%) of the title compound.
¹H NMR (DMSO-d₆): δ 0.84-0.87(3H, t), 1.24-1.29(24H, m), 1.38-1.43(9H, m), 1.45-1.55(2H, m), 3.36-3.62(2H, m), 3.67-3.74(1H, m), 4.37-4.45(2H, m)
ES-MS m/z: 480.44 [M+H]⁺

### Example 1-5: Preparation of (4S,5R)-4-((S)-1-amino-2-hydroxyethyl)-2-hydroxy-5-tetradecyl-1,3,2-dioxaphosphoran-2-oxide hydrochloride (1a-1)

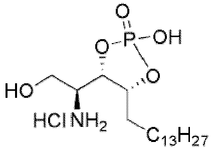

6 g (12.511 mmol) of t-butyl ((1S)-2-hydroxy-1-((4S,5R)-2-hydroxy-2-oxido-5-tetradecyl-1,3,2-dioxaphosphoran-4-yl)ethyl)carbamate (8-1) obtained in Example 1-4 above was dissolved in 120 mL of ethyl acetate, 31 mL of 4M hydrochloride (in dioxane, 125.107 mmol) was added, and the mixture was stirred at 20-25 °C for 18 hours. After the reaction was completed, the reaction solution was concentrated. After adding 120 mL of acetonitrile to the concentrated residue and crystallizing, the mixture was stirred for 3 hours. The resulting crystals were filtered and dried under vacuum to give 4.5 g (86%) of the title compound.
¹H NMR (DMSO-d₆): δ 0.84-0.87(3H, t), 1.16-1.24(24H, m), 1.44-1.68(2H, m), 3.65-3.81(2H, m), 3.93-4.00(1H, m), 4.43-4.48(2H, m)
³¹P NMR (DMSO-d₆): δ -8.03 ppm
¹³C NMR (DMSO-d₆): δ 14.40(1C, s), 22.55(1C, s), 24.92(1C, s), 29.16(1C, s), 29.48-29.64(9C, s), 31.75(1C, s), 46.70(1C, s), 64.93(1C, s), 69.96(1C, s), 80.94(1C, s)
ES-MS m/z: 380.39 [M+H]⁺

### Example 2: Preparation of (4S,5R)-4-((S)-1-amino-2-hydroxyethyl)-2-methoxy-5-tetradecyl-1,3,2-dioxaphosphorane-2-oxide hydrochloride (1a-2)

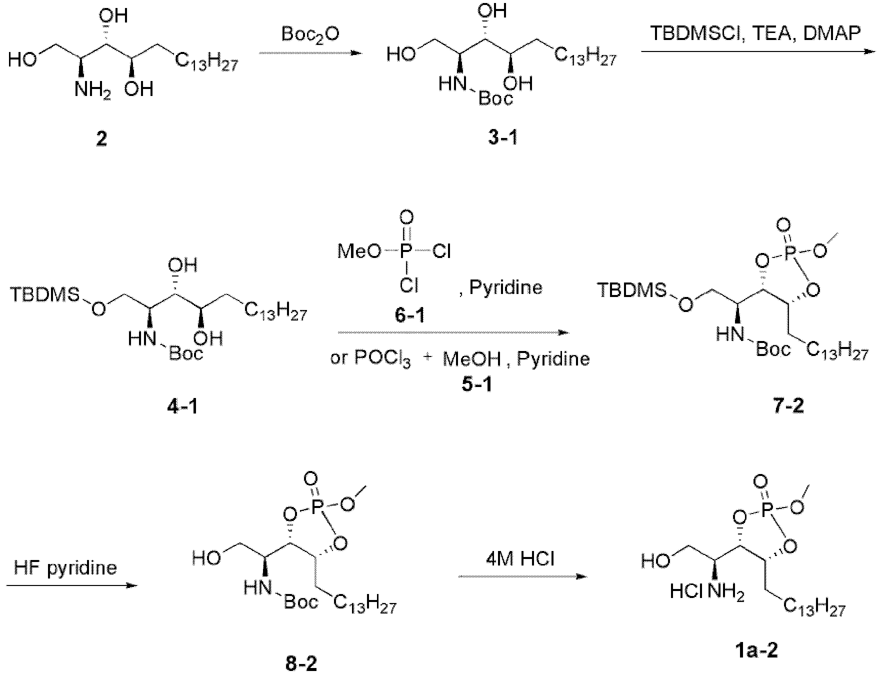

### Example 2-1: Preparation of t-butyl ((1S)-2-((t-butyldimethylsilyl)oxy)-1-((4S,5R)-2-methoxy-2-oxido-5-tetradecyl-1,3,2-dioxaphosphoran-4-yl)ethyl)carbamate (7-2)

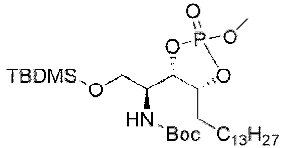

10 g (18.801 mmol) of t-butyl ((2S,3S,4R)-1-((t-butyldimethylsilyl)oxy)-3,4-dihydroxyoctadecan-2-yl)carbamate (4-1) obtained in Example 1-2 above was dissolved in 100 mL of dichloromethane and then cooled to 0 °C. After adding 6.1 mL (75.205 mmol) of pyridine, 2.4 mL (24.442 mmol) of methyl phosphorodichloridate diluted in 20 mL of dichloromethane was slowly added dropwise. The mixture was stirred at 20-25 °C for 3 hours, and the compound was concentrated after the reaction was completed. 100 mL of ethyl acetate (EA) was added to the concentrated compound, stirred, and the resulting crystals were filtered. The filtrate was concentrated under reduced pressure to give 4.8 g (48%) of the title compound.
¹H NMR (DMSO-d₆): δ 0.87-0.91(12H, m), 1.25-1.30(24H, m), 1.43-1.46(9H, m), 1.55-1.63(2H, m), 3.44-3.52(2H, m), 3.62(3H, s) 3.68-3.75(1H, m), 4.43-4.52(2H, m)
ES-MS m/z: 608.85 [M+H]⁺

### Example 2-1-1: Preparation of t-butyl ((1S)-2-((t-butyldimethylsilyl)oxy)-1-((4S,5R)-2-methoxy-2-oxido-5-tetradecyl-1,3,2-dioxaphosphoran-4-yl)ethyl)carbamate (7-2)

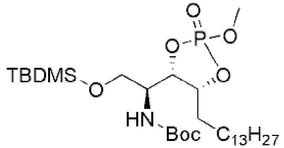

10 g (18.801 mmol) of t-butyl ((2S,3S,4R)-1-((t-butyldimethylsilyl)oxy)-3,4-dihydroxyoctadecan-2-yl)carbamate (4-1) prepared in Example 1-2 above was dissolved in 100 mL of dichloromethane and then cooled to 0 °C. After adding 6.1 mL (75.205 mmol) of pyridine, 2.3 mL (24.442 mmol) of phosphoryl trichloride diluted in 20 mL of dichloromethane was slowly added dropwise. The mixture was stirred at 20-25 °C for 3 hours, and 10 mL of methanol was added after the reaction was completed. After 2 hours, the compound was concentrated, 100 mL of ethyl acetate (EA) was added and stirred, and the resulting crystals were filtered. The filtrate was concentrated under reduced pressure to give 5.7 g (55%) of the title compound.
¹H NMR (DMSO-d₆): δ 0.89-0.92(12H, m), 1.26-1.30(24H, m), 1.43-1.49(9H, m), 1.53-1.62(2H, m), 3.45-3.53(2H, m), 3.64(3H, s) 3.69-3.76(1H, m), 4.41-4.52(2H, m)
ES-MS m/z: 608.85 [M+H]⁺

### Example 2-2: Preparation of t-butyl ((1S)-2-hydroxy-1-((4S,5R)-2-methoxy-2-oxido-5-tetradecyl-1,3,2-dioxaphosphoran-4-yl)ethyl)carbamate (8-2)

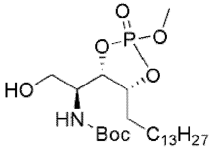

4.8 g (7.896 mmol) of t-butyl ((1S)-2-((t-butyldimethylsilyl)oxy)-1-((4S,SR)-2-methoxy-2-oxido-5-tetradecyl-1,3,2-dioxaphosphoran-4-yl)ethyl)carbamate (7-2) obtained in Example 2-1 above was dissolved in 50 mL of tetrahydrofuran, 0.84 mL (31.584 mmol) of hydrogen fluoride pyridine was added, and the mixture was stirred at 20-25 °C for 16 hours. After the reaction was completed, 3.3 g (31.584 mmol) of sodium carbonate was added and stirred for 3 hours. After adding 50 mL of 1N hydrochloric acid to the reaction solution, 100 mL of ethyl acetate was added to perform extraction. The separated organic layer was dried over anhydrous magnesium sulfate and concentrated under reduced pressure to give 2.5 g (64%) of the title compound.
¹H NMR (DMSO-d₆): δ 0.84-0.86(3H, t), 1.22-1.30(24H, m), 1.37-1.43(9H, m), 1.46-1.55(2H, m), 3.36-3.60(2H, m), 3.61(3H, s), 3.67-3.71(1H, m), 4.38-4.47(2H, m)
ES-MS m/z: 494.12 [M+H]⁺

### Example 2-3: Preparation of (4S,5R)-4-((S)-1-amino-2-hydroxyethyl)-2-methoxy-5-tetradecyl-1,3,2-dioxaphosphoran-2-oxide hydrochloride (1a-2)

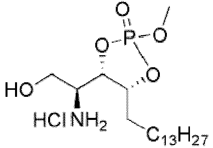

2.5 g (5.064 mmol) of t-butyl ((1S)-2-hydroxy-1-((4S,5R)-2-methoxy-2-oxido-5-tetradecyl-1,3,2-dioxaphosphoran-4-yl)ethyl)carbamate (7-2) obtained in Example 2-2 above was dissolved in 50 mL of ethyl acetate, then 12.7 mL of 4M hydrochloride (in dioxane, 50.647 mmol) was added, and the mixture was stirred at 20-25 °C for 18 hours. After the reaction was completed, the reaction solution was concentrated. To the concentrated residue was added 100 mL of acetonitrile to crystallize, and the mixture was stirred for 3 hours. The resulting crystals were filtered and vacuum-dried to give 1.9 g (90%) of the title compound.
¹H NMR (DMSO-d₆): δ 0.85-0.87(3H, t), 1.15-1.25(24H, m), 1.43-1.67(2H, m), 3.61(3H, s), 3.66-3.81(2H, m), 3.95-4.01(1H, m), 4.43-4.46(2H, m)
³¹P NMR (DMSO-d₆): δ -8.01 ppm
¹³C NMR (DMSO-d₆): δ 14.40(1C, s), 22.55(1C, s), 24.92(1C, s), 29.16(1C, s), 29.48-29.64(9C, s), 31.75(1C, s), 46.70(1C, s), 55.3(1C, s), 64.93(1C, s), 69.96(1C, s), 80.94(1C, s)
ES-MS m/z: 393.56 [M+H]⁺

### Experimental Example 1: Analyzing the effect on SIRT1 expression

To determine the increase in SIRT1 expression by the compound of formula (1a-1) prepared in Example 1 above in human umbilical vein endothelial cells (HUVECs), HUVECs were seeded at 5 × 10⁵/4 ml in a 60 mm cell culture dish using ECBM2 medium (Promocell) and stabilized for 12 hours in an incubator maintained at 37°C and 5% CO₂, respectively.

The cells were then treated with 10 nM, 100 nM and 1000 nM of the compound of formula (1a-1) prepared in Example 1 above, respectively, and incubated for another 24 hours, and then SIRT1 expression was determined by western blot.

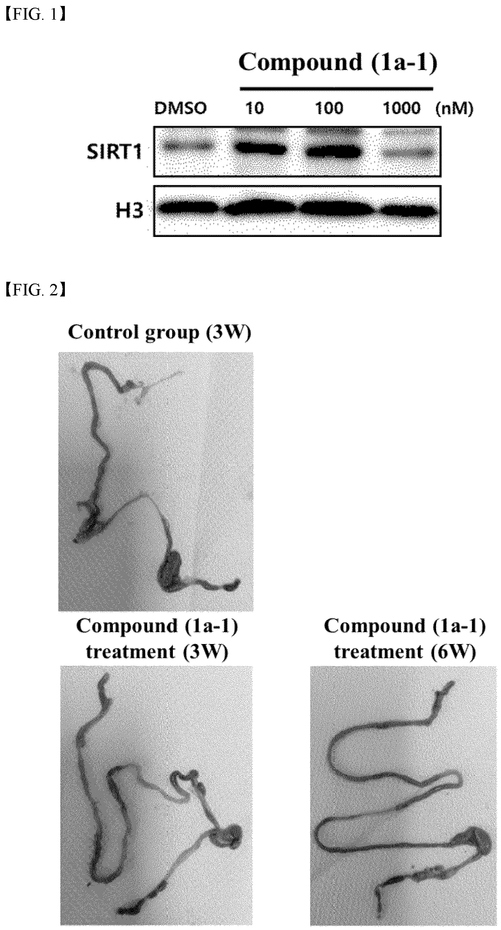

The results are shown in FIG. 1.

From FIG. 1, it can be seen that treatment of HUVECs with the compound of formula (1a-1) increases the expression of SIRT1 even at a very low concentration of 10 nM. In particular, the compound of formula (1a-1) showed a concentration (EC₅₀) of about 10 nM that increased the expression of SIRT1 by 50%.

On the other hand, when the compound of formula (1a-1) was treated to mouse vascular cells, it was shown to exhibit maximum expression at 100 ng/mL.

In the case of resveratrol, which is well known as a SIRT1 activator, the EC₅₀ is known to be approximately 36 µM. Therefore, it can be confirmed that the compound of formula (1a-1) increases the expression of SIRT1 at a concentration thousands of times lower than that of resveratrol.

### Experimental Example 2: Analysis of changes in intestinal length in an AD-induced animal model

In neurodegenerative diseases (AD and PD), the length of the intestine tends to decrease as the disease progresses, so we looked for changes in intestinal length in an AD-induced animal model.

APP/PS/Tau transgenic mice were used as an AD-induced animal model. The compound of formula (1a-1) was administered intraperitoneally once every 3 days at a concentration of 1 mg/kg to 12-week-old transgenic mice for 2 weeks, and the changes in intestinal length of the mice were measured after 1 and 4 weeks.

The results are shown in FIG. 2.

Referring to FIG. 2, it can be seen that the intestinal length is increased in the group of mice treated with the compound of formula (1a-1) compared to the AD-induced mice in the control group that did not receive the compound of formula (1a-1).

In particular, when examining the length of the intestine at 1 week (3W) and 4 weeks (6W) after administration of the compound of formula (1a-1), it can be seen that the length of the intestine at 4 weeks is longer compared to the length of the intestine at 1 week.

### Experimental Example 3: Analyzing drug efficacy in an AD-induced animal model

To determine the therapeutic effect on Alzheimer's Disease (AD), the effects on amyloid beta monomer (monomeric Aβ, mAβ) and Tau protein, which are biomarkers of AD, were analyzed in an AD-induced animal model.

APP/PS/Tau transgenic mice were used as an AD-induced animal model. The compound of formula (1a-1) was administered intraperitoneally once every 3 days at a concentration of 1 mg/kg to 12-week-old transgenic mice for 2 weeks, and the expression of mAβ and Tau proteins in the mouse brain was measured after 1 and 4 weeks. After anesthetizing the mouse, the cervical region was incised with a dissecting tool, and then the occipital region was incised to remove both sides of the skull. The brain was washed with phosphate buffered saline (PBS) and fixed with 4% paraformaldehyde (Sigma, St Louis, MO, USA) for 12 hours at 4°C. These were cut to a thickness of approximately 10 µm using a vibratome, and hippocampus and cortex sections were collected and placed in 24 wells. PBS was added to the wells containing the fixed tissue sections, and fluorescent nanoparticles (QD565, red fluorescence) conjugated with anti-amyloid antibodies and anti-Tau antibodies (Santa Cruz, USA) were added to each 2 µM, and reacted at 4°C for 12 hours. The expression of mAβ and Tau was confirmed by fluorescence microscopy.

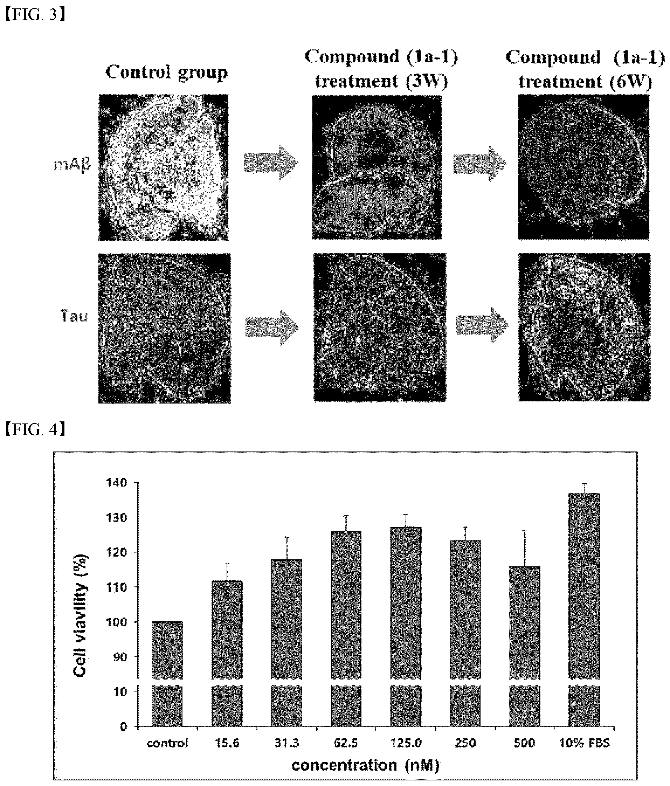

The results are shown in FIG. 3.

From FIG. 3, it can be seen that the mice not treated with the compound of formula (1a-1) showed very strong red fluorescence for mAβ and Tau, while the mice treated with the compound of formula (1a-1) showed negligible red fluorescence for mAβ and Tau at 1 week and 4 weeks after drug administration. Therefore, it can be seen that the expression of amyloid and Tau proteins, which are biomarkers of Alzheimer's disease, is significantly reduced when the compound of formula (1a-1) is administered. These results indicate that the compound of formula (1a-1) can be used as a treatment for Alzheimer's disease.

### Experimental Example 4: Analyzing the effect on proliferation of human neurons

The SH-SY5Y cell lines derived from human neuroblastoma, which exhibit characteristics similar to dopaminergic neurons, were used as a Parkinson's disease (PD) cell model to evaluate the proliferative efficacy of neurons.

A medium containing DMEM/F12 (Hyclone), 10% FBS (Hyclone), and 1% penicillin-streptomycin (Hyclone) was used, and the medium was replaced every two days after seeding on a T75 plate. Subculture was performed when the cell density was 80-90%.

Then, to determine the neuronal proliferation effect, the subcultured SH-SY5Y cells were seeded in 96 wells at 5 × 10³ cells/well. After 1 day, the medium was replaced with a 5% FBS ratio, and the cells were treated with the compound of formula (1a-1) at concentrations serially diluted 2-fold from 500 nM to 15.6 nM and cultured for 5 days. The medium was replaced with 10% FBS containing the drug every other day. The culture medium with 10% FBS was used as a control. After 5 days, the amount of living cells was measured at 450 nm absorbance using EZ-cytox (DoGen) to compare the cell viability.

The results are shown in FIG. 4.

From FIG. 4, it can be seen that the proliferation of nerve cells increases with increasing concentrations of the compounds of formula (1a-1), showing maximum proliferative potency near 100 nM.

### Experimental Example 5: Analyzing the effect on differentiation into neurons

Pluripotent stem cells were seeded in AggreWell plates (stemcell) at 5,000 cells/microwell and cultured for 5 days in E6 medium (GIBCO) to induce embryonic body (EB) formation. For neural rosette formation, EBs were seeded into 35 mm dishes in groups of 30 and cultured for 10 days in N2bF medium. The 35 mm dishes were used after being coated with 15 µg/ml of poly-L-ornithine (sigma) and 1 µg/ml of fibronectin (sigma). The N2Bf medium composition was DMEM/F12 (GIBCO), N2 supplement (stemcell) 1×, 10% NEAA (GIBCO), 55 µM betaMercaptoethanol (GIBCO), bFGF (peprotech) 20 ng/ml. For the formation of spherical neural masses (SNMs), rosette masses were sliced and cultured in petri dishes with N2bF medium. SNMs were no larger than 500 µM in size and were sliced and cultured for 3 to 4 passages. After subculture of SNM, the clumps were broken into small pieces using a tungsten mesh (Nilaco) for the neural progenitor cell (NPC) culture step, and then attached to a PLO/FN-coated dish and cultured in N2bF medium. To differentiate NPCs into dopaminergic neurons, the medium was replaced with an ITS-B27 medium after 2 days of NPC culture. The ITS-B27 medium composition was DMEM/F12 (GIBCO), D-(+)-glucose (sigma) 1.5 mg/ml, insulin (wako) 5 µg/ml, transferrin (wako) 50 µg/ml, selenite (sigma) 30 nM, GlutaMAX (GIBCO) 2.5%, and B27 (GIBCO) 1×. After 4 days of NPC culture, 200 ng/ml of Sonic Hedgehog and 100 ng/ml of FGF8 (peprotech) were added to the ITS-B27 medium. After 6 days of NPC culture, 200 ng/ml of Sonic Hedgehog, 20 ng/ml of GDNF, 20 ng/ml of BDNF (peprotech), 200 µM of ascorbic acid, and 500 µM of cAMP (sigma) were added to the ITS-B27 medium and cultured for approximately 5 weeks. The compound of formula (1a-1) was treated starting from the stage 2 days after NPC culture. The case in which differentiation was induced without treatment with the compound of formula (1a-1) was designated as the differentiation control group (differentiated CTL). In addition, for comparison, the case in which the compound of formula (1a-1) was not treated and differentiation was not induced was used as an undifferentiated control group (undifferentiated CTL).

The effect of the compound of formula (1a-1) on differentiation into neurons was evaluated by comparing the expression of tyrosine hydroxylase (TH) and dopamine transporter (DAT) depending on the presence or absence of treatment with the compound of formula (1a-1).

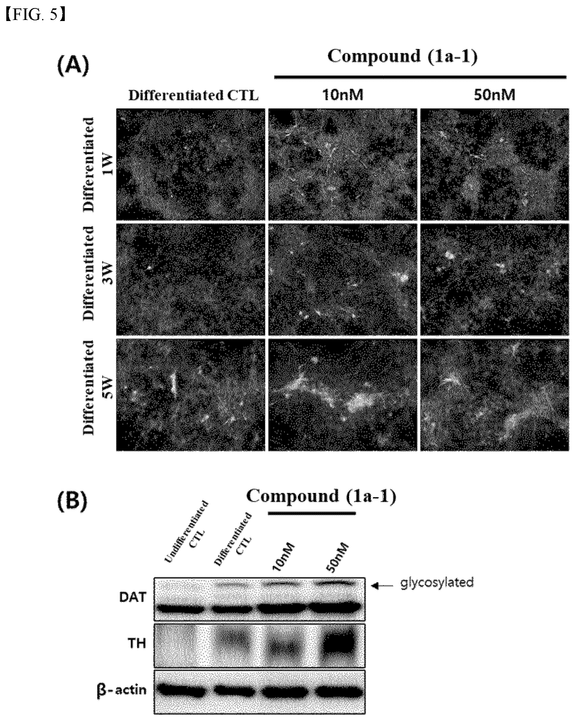

The results are shown in FIG. 5.

FIG. 5A shows the results of cell fluorescence immunostaining, which confirmed that the expression of TH increased upon treatment with the compound of formula (1a-1). In particular, TH expression, which increases after more than 5 weeks of differentiation, was elevated as early as 3 weeks of treatment with the compound of formula (1a-1). FIG. 5B is the results of western blot of a 5-week differentiation sample. It can be seen that TH expression is increased and DAT glycosylation is increased upon treatment with the compound of formula (1a-1). Thus, it can be seen that the compound of formula (1a-1) can promote the differentiation of pluripotent stem cells into dopaminergic neurons.

### Experimental Example 6: Analyzing the effect on neuron-specific protein expression

To analyze the effect on the expression of tyrosine hydroxylase (TH) and α-synuclein as neuron-specific proteins, western blot was performed.

SH-SY5Y cell line was treated with retinoic acid and 12-O-tetradecanoylphorbol-13-acetate (TPA) to induce differentiation into TH-secreting neurons for 6 days.

Differentiated dopaminergic neurons were lysed in PRO-PREP (Intronbio), a cell lysis solution containing protease inhibitor cocktail (Gendepot), and electrophoresed in 10% SDS polyacrylamide gels at 20 µg/well. Transfer was performed using PVDF (Merck millipore) as a membrane, and tyrosine hydroxylase antibody, α-synuclein antibody (Cell signaling), and β-actin antibody (Santa Cruz) were used for reaction for more than 2 hours. Protein expression was detected by staining with ECL substrate (Biorad) for development and graphed using the image J program.

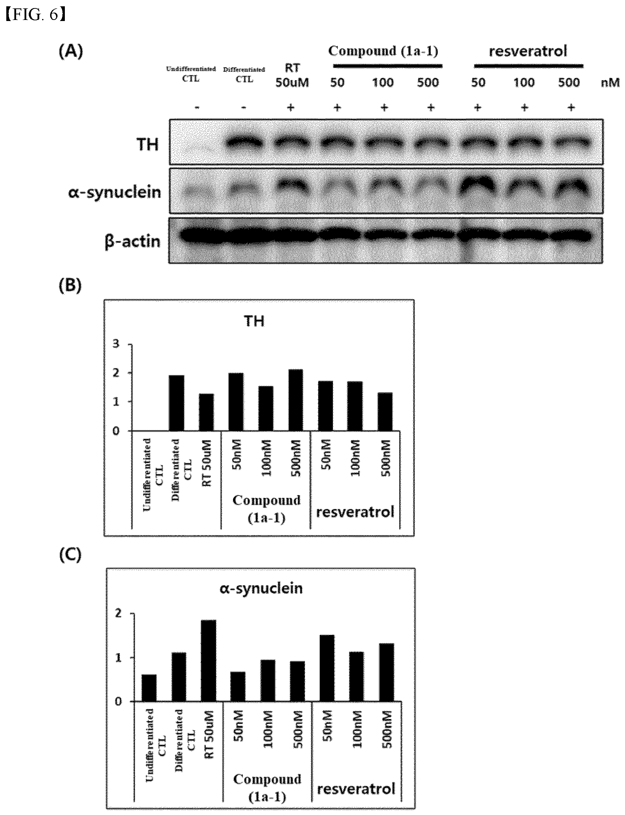

The results are shown in FIG. 6.

Specifically, FIG. 6A shows western blot results measuring changes in the expression of tyrosine hydroxylase and α-synuclein upon treatment with the compound of formula (1a-1), and FIGS. 6B and 6C are graphs depicting western blot results measuring changes in the expression of tyrosine hydroxylase and α-synuclein, respectively.

From FIG. 6, it can be seen that the expression of TH is significantly increased in differentiated dopaminergic neurons (differentiated CTLs) compared to undifferentiated neurons, SH-SY5Y cell line (undifferentiated CTLs), but when the differentiated dopaminergic neurons are treated with rotenone (RT), the expression of TH is decreased and the expression of α-synuclein is increased. However, upon treatment with the compound of formula (1a-1), the expression of TH decreased by rotenone was restored, and conversely, the expression of α-synuclein increased by rotenone was decreased. In particular, the compound of formula (1a-1) was more effective than resveratrol, which was used as a control drug.

Rotenone is known as a drug inducing Parkinson's disease. Therefore, the above results show that the compound of formula (1a-1) can be used as a treatment for Parkinson's disease.

### Experimental Example 7: Analyzing the effects on sepsis induced by CLP treatment

C57BL/6 mice aged 7-8 weeks were purchased from Jabio (Suwon, Gyeonggi-do) and sepsis was induced by cecal ligation and puncture (CLP).

Specifically, after anesthesia using 150 mg/kg of ketamine and 17.5 mg/kg of rompun, an abdominal incision of 1-2 cm was made to expose the cecum to induce sepsis. The cecum was ligated with 6-0 silk sutures at the distal end of the ileocecal valve, and a puncture was made with an 18-gauge needle. The perforated cecum was gently squeezed to push out 1-2 cm of feces, and the cecum was then retracted back into the abdominal cavity. The abdomen was then closed and pre-warmed normal saline (2.5 mL/100 g body weight) was injected into the mouse abdominal cavity immediately after the procedure. 6 and 16 hours after CLP treatment, the compound of formula (1a-1) was dissolved in phosphate buffered saline (PBS) and injected intravenously at 100 ng/mouse (5 µg/kg), and survival was examined for 10 days. For comparison, as a surgical-negative control (sham control), survival was examined in mice that did not receive CLP treatment but underwent the same open surgery. In addition, as a drug-negative control, survival was examined in mice that received only PBS after CLP treatment.

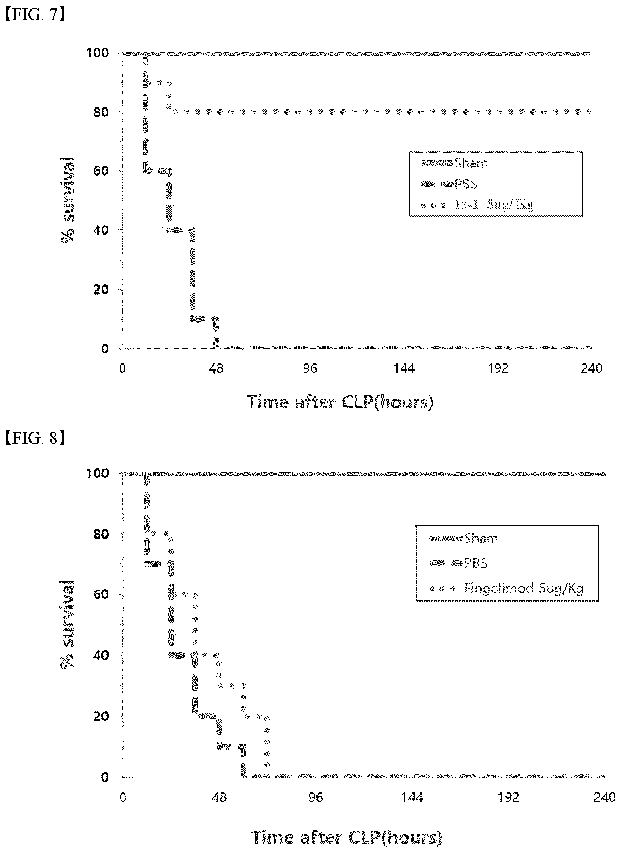

The results are shown in FIG. 7.

From FIG. 7, it can be seen that all individuals in the drug-negative control group, which received only PBS, die within 48 hours. Thus, it can be seen that the above CLP treatment condition is a very high level severe sepsis model.

In this severe sepsis model, the experimental group treated with the compound of formula (1a-1) at 6 and 16 hours after CLP treatment showed more than 80% survival for 10 days. There were no deaths in the experimental group after 10 days. Therefore, it can be seen that the compound of formula (1a-1) is effective in the treatment of severe sepsis.

For comparison, we also examined the survival of a control group treated with fingolimod (FTY720), a known S1P modulator, instead of the compound of formula (1a-1) under the same conditions.

The results are shown in FIG. 8.

From FIG. 8, it can be seen that FTY720 did not improve survival.

### Experimental Example 8: Analyzing the effect on acute respiratory distress syndrome induced by CLP treatment

CLP induces pulmonary edema due to increased inflammation and disruption of the vascular barrier, resulting in acute respiratory distress syndrome (ARDS). To determine the effect on ARDS, the compound of formula (1a-1) was treated at 6 and 16 hours after CLP treatment in the same manner as in Experimental Example 7 above, the lungs of mice were harvested at 18 hours for histological analysis, and the wet-to-dry weight ratio of the lungs was obtained. For comparison, mice without CLP treatment served as a control group.

For histologic analysis, the harvested lungs were fixed in 10% paraformaldehyde, infiltrated with paraffin, sectioned into 5 µm-thick tissues, and then de-paraffinized with xylene. Sectioned tissues were mounted on slides, stained with H&E, and observed under an optical microscope. In addition, the lung tissue damage was evaluated by histological score to obtain the lung injury score (LIS) for each group.

To calculate the wet-to-dry weight ratio, lung organs were harvested 18 hours after CLP treatment, and the wet weight was measured immediately after harvesting, and the dry weight was measured after drying in an oven at 60°C for 48 hours to calculate the wet-to-dry weight ratio. The wet-to-dry weight ratio is used as an indicator of organ edema formation.

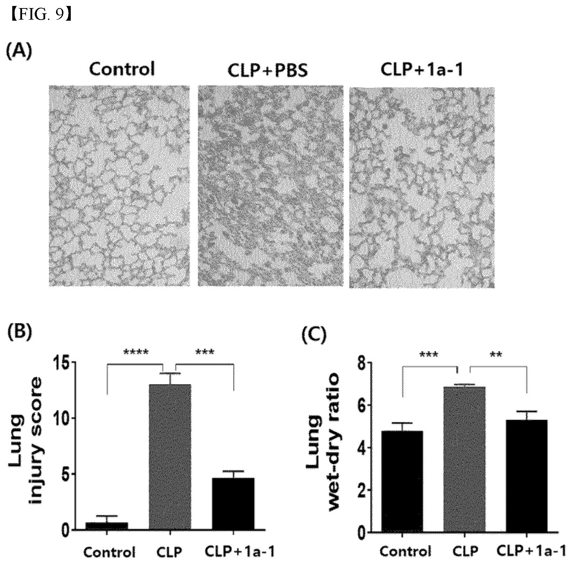

The results of the histologic analysis and wet-to-dry weight ratio measurement are shown in FIG. 9.

FIG. 9A is a photograph of lung tissue observed under an optical microscope when treated with the compound of formula (1a-1) after CLP treatment, FIG. 9B is a graph showing the results of the lung injury score, which evaluates lung tissue damage using a histological score, and FIG. 9C is a graph showing the wet-to-dry weight ratio of the harvested lungs.

From FIG. 9A, it can be seen that the lung tissue is damaged after CLP treatment, and the lung tissue is almost restored to normal when treated with the compound of formula (1a-1). Furthermore, FIG. 9B shows that the lung injury score is significantly elevated after CLP treatment, but treatment with the compound of formula (1a-1) almost restores the lung injury score to normal. Furthermore, FIG. 9C shows that after CLP treatment, the wet-to-dry weight ratio of the lung increases as the lung tissue becomes water-logged with pulmonary edema due to inflammation and vascular barrier disruption, but treatment with the compound of formula (1a-1) restores the wet-to-dry weight ratio of the lung to normal.

Therefore, it can be seen that the compound of formula (1a-1) can treat ARDS.

### Experimental Example 9: Analyzing the effect on inflammatory cytokines after CLP treatment

After CLP treatment, the inflammatory cytokines TNF-α and IL-6 were analyzed by ELISA.

Specifically, after treating the compound of formula (1a-1) at 6 and 16 hours after CLP treatment, mouse blood was collected at 18 hours and allowed to stand at room temperature for 1 hour, then centrifuged at 3000 rpm for 10 minutes to obtain serum to analyze the amount of TNF-α and IL-6.

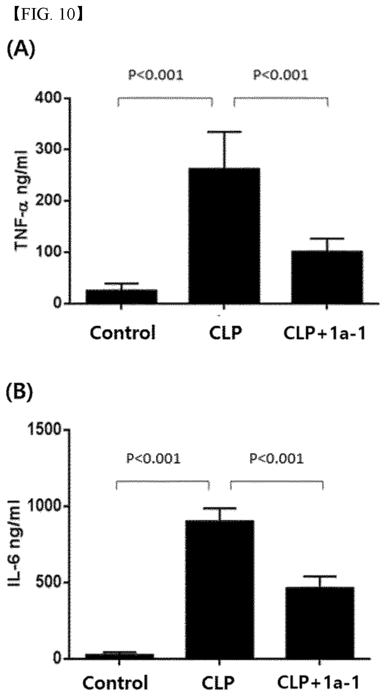

The results are shown in FIG. 10.

FIG. 10 is a graph showing the effect of treatment with the compound of formula (1a-1) on the secretion of the inflammatory cytokines TNF-α (A) and IL-6 (B) after CLP treatment.

From FIG. 10, it can be seen that the amount of inflammatory cytokines TNF-α and IL-6 is significantly increased in the CLP-treated control group, but the amount of inflammatory cytokines is significantly decreased by treatment with the compound of formula (1a-1).

Thus, it can be seen that the compound of formula (1a-1) has an excellent ability to inhibit the inflammatory response, which is important in the treatment of sepsis or ARDS.

### Experimental Example 10: Analyzing the effect on permeability

Permeability was measured by an in vitro method using cell culture and in vivo method using Evans blue dye. To increase permeability, LPS was used in the in vitro method and CLP treatment was used in the in vivo method.

To measure permeability by an in vitro method, MYSEC (murine yolk sac endothelial cell) cell lines were used. MYSEC cell lines were placed in the top chamber of a trans well at 2.5×10⁵ cells/well and treated with 300 ng of lipopolysaccharide (LPS) derived from Gram-negative bacteria in the presence of the compound of formula (1a-1) for 24 hours. The medium in the top chamber was replaced with 300 µl of serum-free medium containing 5 µl of streptoavidin-HRP. After 5 minutes, 20 µl of the medium in the lower chamber was taken and the activity of HRP was measured as absorbance at 450 nm.

To determine the mechanism by which the compound of formula (1a-1) improves permeability, the same experiment was performed by knocking down the SIRT1 protein in the MYSEC cell line.

To measure permeability by an in vivo method, the compound of formula (1a-1) was treated at 6 and 16 hours after CLP treatment in the same manner as in Experimental Example 7 above, and 200 µl of a 0.5% Evans blue dye (EBD) solution was administered intravenously at 18 hours, circulated for 1 hour, and organs were harvested to visually confirm the presence of dye. The organs were then homogenized in a homogenizer to extract the dye at 55 °C for 48 hours, and permeability was determined by measuring absorbance at 620 nm.

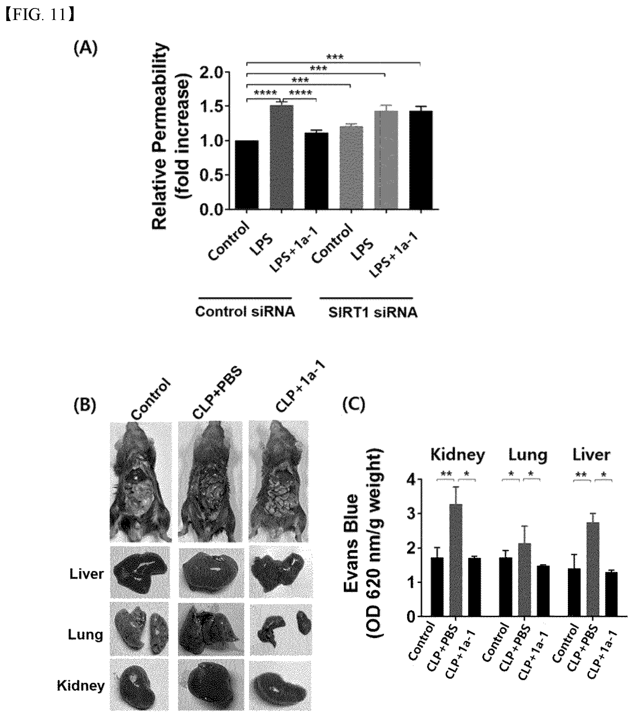

The results are shown in FIG. 11.

FIG. 11A is a graph showing the effect on the permeability of the MYSEC cell line upon LPS treatment in the presence of the compound of formula (1a-1), and FIGS. 11B and 11C are a photograph and a graph showing the effect on the permeability of mouse organs upon treatment with the compound of formula (1a-1) after CLP treatment, respectively.

From FIG. 11A, it can be seen that treatment of the MYSEC cell line with LPS increases permeability, but treatment with the compound of formula (1a-1) restores the LPS-induced increased permeability to normal. However, knockdown of the SIRT1 protein in the MYSEC cell line does not show the effect of the compound of formula (1a-1). Therefore, it can be confirmed that the compound of formula (1a-1) improves permeability by increasing the expression of SIRT1.

From FIG. 11B, it can be seen that induction of CLP leads to the collapse of the vascular barrier, and a large amount of EBD is distributed in various organs.

Furthermore, in FIG. 11C, it can be seen that induction of CLP increases the amount of EBD in each organ, but it returns to normal upon treatment with the compound of formula (1a-1).

Thus, it can be seen that the compound of formula (1a-1) can ameliorate sepsis and ARDS by strengthening the vascular barrier.

## Claims

1. A compound of formula (1) or pharmaceutically acceptable salt thereof: 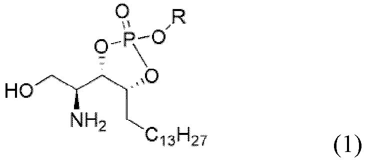 wherein,
R is hydrogen, a C₁-C₆ alkyl or an aryl.

2. The compound according to claim 1 or pharmaceutically acceptable salt thereof, wherein R is hydrogen or a C₁-C₆ alkyl.

3. The compound according to claim 1 or pharmaceutically acceptable salt thereof, wherein the pharmaceutically acceptable salt is a hydrochloride salt.

4. A method for preparing a compound of formula (1) or pharmaceutically acceptable salt thereof, the method comprising:
(i) protecting an amine group of a compound of formula (2) to obtain a compound of formula (3);
(ii) protecting a primary hydroxyl group of the compound of formula (3) to obtain a compound of formula (4);
(iii) reacting the compound of formula (4) with phosphoryl chloride (POCl₃), or phosphoryl chloride (POCl₃) and a compound of formula (5), or a compound of formula (6), to obtain a compound of formula (7);
(iv) selectively deprotecting the hydroxyl protecting group of the compound of formula (7) to obtain a compound of formula (8); and
(v) deprotecting the amine protecting group of the compound of formula (8): 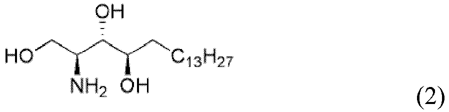 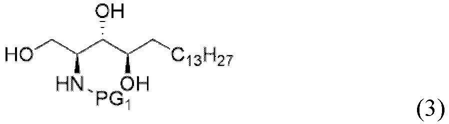 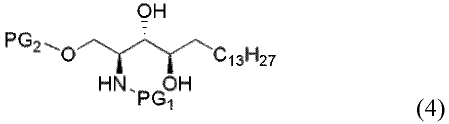
R'-OH (5) 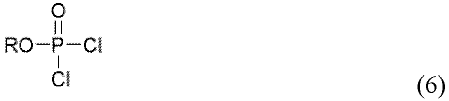 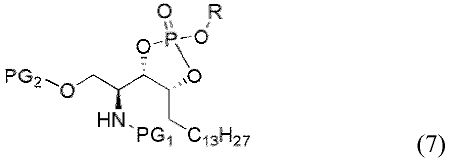 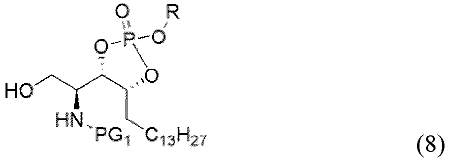 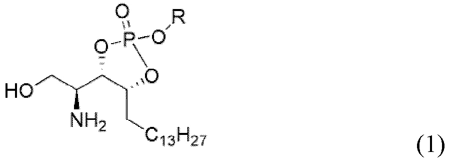
wherein,
PG₁ is an amine protecting group;
PG₂ is a hydroxyl protecting group;
R' is a C₁-C₆ alkyl or an aryl; and
R is hydrogen, a C₁-C₆ alkyl or an aryl.

5. The method according to claim 4, wherein PG₁ is t-butyloxycarbonyl.

6. The method according to claim 4, wherein PG₂ is t-butyldimethylsilyl.

7. The method according to claim 4, wherein the deprotection is performed in the presence of a base and HF in step (iv).

8. The method according to claim 4, wherein the deprotection is performed using an acid in step (v).

9. A pharmaceutical composition for preventing or treating a neurodegenerative disease comprising the compound according to any one of claims 1 to 3 or pharmaceutically acceptable salt thereof.

10. The pharmaceutical composition according to claim 9, wherein the neurodegenerative disease is Alzheimer's disease or Parkinson's disease.

11. A pharmaceutical composition for preventing or treating sepsis or acute respiratory distress syndrome comprising the compound according to any one of claims 1 to 3 or pharmaceutically acceptable salt thereof.

12. A dietary supplement for preventing or ameliorating a neurodegenerative disease comprising the compound according to any one of claims 1 to 3 or pharmaceutically acceptable salt thereof.

13. The dietary supplement according to claim 12, wherein the neurodegenerative disease is Alzheimer's disease or Parkinson's disease.
